# EUROPÄISCHE PATENTSCHRIFT

(11) **EP 1 938 790 B1**
(45) Veröffentlichungstag und Bekanntmachung des Hinweises auf die Patenterteilung: **29.06.2011**
(21) Anmeldenummer: 07122537.9
(22) Anmeldetag: 06.12.2007
(51) Int. Cl.: A61K 8/35, A61K 8/55, A61K 8/60, A61K 8/67, A61K 8/97, A61K 8/98, A61Q 19/04, A61K 8/04

(54) **Hautbräunungsmittel auf der Basis von Dihydroxyaceton**
Dihydroxyacetone-based skin tanning lotion
Agent de brunissement de la peau à base de dihydroxyacétone

(30) Priorität: 07.12.2006 DE 102006058394
(43) Veröffentlichungstag der Anmeldung: 02.07.2008
(73) Patentinhaber: Coty Germany GmbH, 55116 Mainz (DE)
(72) Erfinder: Golz-Berner, Karin, 98000 Monaco (MC); Zastrow, Leonhard, 98000 Monaco (MC)
(74) Vertreter: Ziebig, Marlene

(56) Entgegenhaltungen:
- EP-A1- 1 479 374
- WO-A1-02/00214
- WO-A1-99/48386
- WO-A1-2004/105706
- WO-A1-2005/025531
- WO-A1-2006/045760
- WO-A1-2006/066741
- WO-A1-2007/012356
- WO-A1-2007/118565
- WO-A2-99/66881
- DE-A1- 19 603 018
- DE-A1- 19 739 349
- DE-A1- 19 827 624
- FR-A1- 2 793 686
- MERCK: "DHA Rapid speeds up the self-tanning process" INTERNET CITATION 15. Mai 2006 (2006-05-15), Seite 1, XP007913552 Gefunden im Internet: URL:http://me.merck.de/EMD/UK/UKNEWS2.NSF/ d4c60a303233fb87c1256fc50036831 2/3fb30db3921b78bdc125716f002f3c62?OpenDoc ument [gefunden am 2010-06-22]
- EMD CHEMICALS: "RonaCare TM VTA Protector of the Extra Cellular Matrix" 20021201, 1. Dezember 2002 (2002-12-01), Seiten 1-12, XP007913556

## Beschreibung

Die Erfindung betrifft ein neues Hautbräunungsmittel auf Basis von Dihydroxyaceton (DHA) mit verbesserter Bräunungswirkung, welches eine Basisformulierung umfasst, die DHA, Trihydroxyethylrutin, Rutinylsulfat und mindestens ein weiteres Antioxidationsmittel enthält.

DHA ist in der Kosmetik ein anerkanntes Hautbräunungsmittel. Seine Wirkung ist jedoch nicht immer befriedigend, weshalb verschiedene Varianten mit verbessernden Zusätzen entwickelt worden sind. Durch Zusatz von Natriumdisulfit wird beispielsweise die Stabilität der Formulierung verbessert, wobei sich allerdings bestimmte Applikationsgrenzen ergeben, z.B. 6% DHA maximal für Gesichtsanwendungen. Weiterhin bekannt aus WO 2005/025531 ist eine Kombination von lamellar eingelagertem DHA und freiem DHA im Verhältnis 1:1,5-1:0,25 zusammen mit Phospholipiden und weiteren kosmetischen Zusatzstoffen, wobei eine bräunungsverlängernde Wirkung beschrieben wird.

Die Kombination von DHA und Troxerutin (Trihydroxyethylrutin) ist bekannt als synergistischer Bräunungsbeschleuniger "DHA Rapid" der Firma Merck KGaA.

Der Erfindung liegt die Aufgabe zugrunde, ein Hautbräunungsmittel zu entwickeln, das eine verbesserte Langzeitwirkung von DHA zeigt und gleichzeitig die Entwicklung freier Radikale einschränkt.

Erfindungsgemäß bereitgestellt wird ein Mittel auf Basis von DHA mit verbesserter Bräunungswirkung, das eine Hautbräunungs-Basisformulierung umfasst, die Dihydroxyaceton, Trihydroxyethylrutin, Rutinylsulfat und mindestens ein weiteres Antioxidationsmittel enthält. Das weitere Antioxidationsmittel ist bevorzugt ausgewählt aus der Gruppe umfassend Vitamin C, Vitamin A, Vitamin E, Derivate von Vitamin C, Derivate von Vitamin A, Derivate von Vitamin E, Gemische der Derivate, Folsäure, Derivate von Folsäure, Flavone, Flavonoide, Aminosäuren, Derivate von Aminosäuren, Carotinoide, Carotine, Harnsäure, Derivate von Harnsäure, α-Hydroxysäuren, Pflanzenextrakte, Gemische von Pflanzenextrakten und Gemische von mehreren Antioxidationsmitteln.

Es wurde gefunden, dass eine durch den Zusatz von Trihydroxyethylrutin auftretende schnellere und etwas höhere Wirksamkeit von DHA durch den Zusatz einer Kombination von Rutinylsulfat und einem weiteren Antioxidationsmittel über einen langen Zeitraum von mehr als 8 Wochen nahezu konstant gehalten werden kann. Eine solche Langzeitwirkung ist nicht zu erwarten gewesen. Der Abbau von DHA, der normalerweise nach etwa 2-4 Wochen langsam beginnt und nach 8 Wochen schon bei 20-25 % liegt, wird hier deutlich gestoppt. Mit der erfindungsgemäßen Bräunungs-Basisformulierung ist keine signifikante Verringerung der Hautbräunung nach Ablauf dieses Zeitraumes zu erkennen.

Weiterhin wird durch den Zusatz von Rutinylsulfat bei gleichzeitiger Anwesenheit von DHA und Trihydroxyethylrutin (Truxerutin) der für DHA typische Gelb-Rot-Farbton vermieden und ein deutlich bräunlicher Farbton erzielt, der für die tägliche Anwendung eines entsprechenden kosmetischen Produktes sehr erwünscht ist.

Der Anteil der Bräunungs-Basisformulierung in einem anwendungsbereiten kosmetischen Bräunungsmittel liegt vorzugsweise im Bereich von 0,5 bis 30 Gew-%, bezogen auf das Gesamtgewicht des Mittels.

Bevorzugt enthält die Basisformulierung 6 bis 24 Gew-% eines Gemisches aus DHA und Trihydroxyethylrutin, vorzugsweise 6 bis 20 Gew-%. Das Verhältnis von DHA : Trihydroxyethylrutin beträgt vorzugsweise 60-80 : 40-20.
Sie enthält ferner bevorzugt 0,5 bis 6 Gew-% Rutinylsulfat, vorzugsweise 0,5 bis 3 Gew-%. Das weitere Antioxidationsmittel enthält sie vorzugsweise von 2 bis 12 Gew-%. Die Gew-% sind bezogen auf das Gesamtgewicht der Basisformulierung. Der Rest bis 100 % ist bevorzugt Wasser.

Die Basisformulierung kann in eine Emulsion, wie O/W-, W/O- oder Mehrfachemulsion oder in ein Gel eingebracht werden. Die Herstellung einer Creme, Lotion, Milch, eines Gels kann auf diese Weise erfolgen.

Bevorzugte Bereiche für den Anteil der Bräunungs-Basisformulierung in einem anwendungsbereiten kosmetischen Bräunungsmittel liegen bei 2 bis 20 Gew-%.

Bevorzugte Bereiche für den Anteil an DHA und Trihydroxyethylrutin zusammen liegen in einem anwendungsbereiten kosmetischen Bräunungsmittel bei 1,5 bis 7 Gew-%.

Bevorzugte Bereiche für den Anteil an Rutinylsulfat in einem anwendungsbereiten kosmetischen Bräunungsmittel liegen bei 0,1 bis 2,0 Gew-%.

Ein kosmetisches Bräunungsmittel enthält weiterhin kosmetische Hilfs- und Trägerstoffe, wie sie üblicherweise in solchen Zubereitungen verwendet werden, z.B. Wasser, Konservierungsmittel, Vitamine, Farbstoffe, Pigmente mit färbender Wirkung, Radikalfänger, Verdickungsmittel, weichmachende Substanzen, feuchthaltende Substanzen, Duftstoffe, Alkohole, Polyole, Elektrolyte, Gelbildner, polare und unpolare Öle, Polymere, Copolymere, Emulgatoren, Wachse, Stabilisatoren.

Zu den kosmetischen Wirkstoffen gehören z.B. anorganische und organische Lichtschutzmittel, Radikalfänger, Feuchthaltemittel, Vitamine, Enzyme, pflanzliche Wirkstoffe, Polymere, Melanin, Antioxidationsmittel, entzündungswidrige natürliche Wirkstoffe, mit Sauerstoff beladene asymmetrische lamellare Aggregate gemäß WO 94/00109; Aufschlussprodukte von Hefen oder pflanzlichen Stoffen, hergestellt durch ein schonendes Ultraschall-Aufschlussverfahren gemäß WO 94/13783, Kaolin sowie mit SiO₂ modifiziertes Kaolin gemäß WO 94/17588.

Zu Antioxidationsmitteln gehören Vitamine wie Vitamin C und Derivate davon, beispielsweise Ascorbylacetate, -phosphate und -palmitate; Vitamin A und Derivate davon; Folsäure und deren Derivate, Vitamin E und deren Derivate, wie Tocopherylacetat; Flavone oder Flavonoide; Aminosäuren, wie Histidin, Glycin, Tyrosin, Tryptophan und Derivate davon; Carotinoide und Carotine, wie z.B. α-Carotin, β-Carotin; Harnsäure und Derivate davon; α-Hydroxysäuren wie Citronensäure, Milchsäure, Apfelsäure sowie Pflanzenextrakte und Gemische davon.

Bevorzugt wird als weiteres Antioxidationsmittel in der Basisformulierung ein Pflanzenextrakt oder ein Pflanzenextraktgemisch verwendet. Ein besonders bevorzugtes Antioxidationsmittel ist ein Gemisch von Pflanzenextrakten, bestehend aus
(a) einem durch Extraktion der Rinde von Quebracho blanco und nachfolgender enzymatischer Hydrolyse gewonnenem Produkt, das wenigstens 90 Gew-% Proanthocyanidin-Oligomere und höchstens 10 Gew-% Gallussäure enthält, wobei der Extrakt in einer an Mikrokapseln gebundenen Wirkstoffkonzentration von 2 Gew-% vorliegt, im Bereich von 1 bis 10 Gew-% liegt;
(b) einem durch Extraktion gewonnenen Seidenraupenextrakt, der das Peptid Cecropine, Aminosäuren und ein Vitamingemisch enthält, wobei der Gehalt von (b) im Bereich von 0,1 bis 10 Gew-% liegt;
(c) einem nichtionischen, kationischen oder anionischen HydroGel oder Gemisch von Hydro-Gelen mit einem Gehalt von (c) im Bereich von 0,1 bis 5 Gew-%;
(d) einem oder mehreren Phospholipiden mit einem Anteil von 0,1 bis 30 Gew-%;
(e) Wasser; und gegebenenfalls Cyclodextrinen. Die Gewichtsangaben sind auf das Gesamtgewicht des Antioxidationsmittels bezogen. Dieses Antioxidationsmittel liegt vorzugsweise im Bereich von 0,1-2 Gew-% im Hautbräunungsmittel vor.
Solche Pflanzenextraktgemische sind dem Fachmann als Komplex bekannt und in WO 99/66881 A2 (z.B. Anspruch 1) beschrieben.

Ein weiteres bevorzugtes Antioxidationsmittel ist ein Gemisch von Pflanzenextrakten auf alkoholischer Basis, bestehend aus 0,1 bis 2 Gew-% Extrakt grüner Kaffeebohnen, 0,1 bis 2 Gew-% Extrakt von Blättern von Camellia sinensis, 0,1 bis 2 Gew-% Extrakt von Pongamia pinnata und 0,1 bis 2 Gew-% Extrakt der Wurzeln von Angelica archangelica und dem Rest bis 100 Gew-% aus einem einwertigen C₂-C₅-Alkohol. Dieses Antioxidationsmittel ist frei von Liposomen oder Mikrokapseln, und die Gewichtsangaben sind auf das Gesamtgewicht des Antioxidationsmittels bezogen. Diese Pflanzenextraktgemische sind dem Fachmann als Komplex bekannt und in WO 2004/105706 A2 (z.B. Anspruch 1) beschrieben.

Als Erweichungsmittel können normalerweise eine Vielzahl von Verbindungen eingesetzt werden, wie Stearylalkohol, Glycerylmonoricinoleat, Glycerylmonostearat, Propan-1,2-diol, Butan-1,3-diol, Cetylalkohol, Isopropylisostearat, Stearinsäure, Isobutylpalmitat, Isopropylmyristat, Isopropylpalmitat, Oleylalkohol, Isopropyllaurat, Decyloleat, Octadecan-2-ol, Isocetylalkohol, Cetylpalmitat, Siliconöle wie Dimethylpolysiloxan, Polyethylenglycol, Lanolin, Kakaobutter, pflanzliche Öle wie Maisöl, Baumwollsamenöl, Olivenöl, mineralische Öle, Butylmyristat, Palmitinsäure usw.

Zu Feuchthaltemitteln gehören Glycerin, Butylenglycol, Propylenglycol und Gemische davon.

Polyole sind ebenfalls mögliche Bestandteile des kosmetischen Bräunungsmittels. Dies sind z.B. Propylenglycol, Dipropylenglycol, Ethylenglycol, Isoprenglycol, Glycerin, Butylenglycole, Sorbitol und Gemische davon.

Das erfindungsgemäße kosmetische Bräunungsmittel kann Wachse enthalten, sofern diese zusammen mit Ölen und Wasser in dem Mittel vorliegen. Die Wachse können ausgewählt werden unter natürlichen pflanzlichen Wachsen, tierischen Wachsen, natürlichen und synthetische Mineralwachsen und synthetischen Wachsen. Dazu gehören beispielsweise Carnaubawachs, Candellilawachs, Ozokerit, Bienenwachs, Montanwachs, Wollwachs, Ceresin, Mikrowachse, Hartparaffin, Petrolatum, Siliconwachs, Polyethylenglycol- oder -glycolesterwachse.

Die für die Erfindung eingesetzten Öle können übliche kosmetische Öle sein, wie ein Mineralöl; hydriertes Polyisobuten; synthetisches oder aus Naturprodukten hergestelltes Squalan; kosmetische Ester oder Ether, die verzweigt oder unverzweigt, gesättigt oder ungesättigt sein können; pflanzliche Öle; Siliconöle oder Gemische zweier oder mehrerer davon.

Besonders geeignet Öle sind beispielsweise Mineralöle, Hydrogenated Polyisobuten, Polyisopren, Squalane, Tridecyltrimellitat, Trimethylpropan-triisostearat, Isodecylcitrat, Neopentylglycol-diheptanoat, PPG-15-stearylether, Calendulaöl, Jojobaöl, Avocadoöl, Macadamianußöl sowie Silikonöle, z.B. flüchtige lineare oder cyclische Silikonöle und/oder nicht flüchtige Silikonelastomere wie z.B. vernetztes Polysiloxan, hoch molekulare Silikonpolyether, Copolymere von Polydimethylsiloxan und Dimyldimethylsiloxan und Gemische davon oder ein Gemisch von mehreren der genannten Öle.

Es ist weiterhin vorteilhaft, den kosmetischen Bräunungsmitteln entsprechende wasser- und/oder öllösliche UVA- oder UVB-Filter oder beide zuzusetzen. Zu vorteilhaften öllöslichen UVB-Filtern gehören 4-Aminobenzoesäure-Derivate wie der 4-(Dimethylamino)-benzoesäure-(2-ethylhexyl)ester; Ester der Zimtsäure wie der 4-Methoxyzimtsäure (2-ethylhexyl)ester, Benzophenon-Derivate wie 2-Hydroxy-4-methoxybenzophenon; 3-Benzylidencampher-Derivate wie 3-Benzylidencampher.

Bevorzugte öllösliche UV Filter sind Benzophenone-3, Butyl-Methoxybenzoylmethane, Octyl Methoxycinnamate, Octyl Salicylate, 4-Methylbenzylidene Camphor, Homosalate und Octyl Dimethyl PABA.

Wasserlösliche UVB-Filter sind z.B. Sulfonsäurederivate von Benzophenon oder von 3-Benzylidencampher oder Salze wie das Na- oder K-Salz der 2-Phenylbenzimidazol-5-sulfonsäure.

Zu UVA-Filtern gehören Dibenzoylmethan-Derivate wie 1-Phenyl-4-(4'-isopropylphenyl)propan-1,3-dion, Butyl Methoxybenzoylmethane oder Menthyl Anthranilate.

Besonders bevorzugt sind Benzophenone-3, Butyl Methoxydibenzoylmethane, Octyl Methoxycinnamate, Octyl Salicylate, 4-Methylbenzylidene Camphor, Homosalate, Octocrylene, Ethylhexyl Methoxycinnamate, Isoamyl-p-Methoxycinnamate, Octyl Dimethyl PABA, Ethylhexyltriazone, Diethylhexyl Butamido Triazone, Ethylhexyl Salicylate, Methylene Bis-Benzotriazolyl Tetramethylbutylphenol, Disodium Phenyl Dibenzimidazole Tetrasulfonate, Bis-Ethylhexyloxyphenol Methoxyphenyl Triazine.

Weiterhin einsetzbar als Sonnenschutzfilter sind anorganische Pigmente auf Basis von Metalloxiden, wie TiO₂, SiO₂, ZnO, Fe₂O₃, ZrO₂, MnO, Al₂O₃, die auch im Gemisch verwendet werden können.

Als oberflächenaktive Mittel können anionische, amphotere, nichtionische oder kationische oberflächenaktive Mittel oder Gemische davon eingesetzt werden. Besonders bevorzugt sind kationische Polymere oder ein Gemisch von anionischen und amphoteren oberflächenaktiven Mitteln.

Die Erfindung soll nachstehend durch Beispiele näher erläutert werden. Alle Angaben erfolgen in Gewichtsprozent, sofern nichts anderes angegeben ist.

### Beispiel 1

| **Basisformulierung I** | |
|---|---|
| Dihydroxyaceton | 14 |
| Troxerutin | 6 |
| Rutinylsulfat | 4 |
| Antioxidationsmittel RPF-Komplex I* | 12 |
| Wasser | q.s. ad 100 |

| | |
|---|---|
| * bestehend aus Wasser, 1 % Gelbildner, 7,5 % Phospholipide, 2 % Quebracho-Extrakt und 1 % Seidenraupen-Extrakt bezogen auf Gesamtgewicht Antioxidationsmittel, (Wirkstoffkomplex gemäß WO 99/66881, wobei ein Gemisch aus Phospholipiden (Phoslipon®), Quebracho-Extrakt und Seidenraupenextrakt mit einem Gel (z.B. Carbomer) bei einer Temperatur von höchstens 45°C vermischt, und anschließend ein weiterer Teil des Gels oder ein anderes Gel (z.B. Guar Propyltriammoniumchlorid) bei Temperaturen unter 45°C zugerührt wurde). | |

Die Herstellung der Basisformulierung erfolgt durch Einbringen der Bestandteile in Wasser unter Rühren bei Umgebungstemperatur (15-25 °C).

### Beispiel 2

### Basisformulierung II

| | |
|---|---|
| Dihydroxyaceton | 10 |
| Troxerutin | 3,2 |
| Rutinylsulfat | 2,4 |
| Antioxidationsmittel RPF-Komplex II** | 8,5 |
| Wasser | q.s. ad 100 |

| | |
|---|---|
| ** bestehend aus 99,8 % Ethanol, 0,2 % Kaffeebohnen-Samenextrakt, 0,2 % Blattextrakt von Camellia sinensis, 0,2 % Angelikawurzel-Extrakt und 0,2 Gew-% Extrakt von Pongamia pinnata bezogen auf Gesamtgewicht Antioxidationsmittel (alkoholisches Pflanzenextraktgemisch gemäß WO 2004/105706). | |

### Beispiel 3

### Bräunungscreme mit Sonnenschutz SPF 15

| **Phase A** | |
|---|---|
| Wasser | q.s. ad 100 |
| Glycerin | 10 |
| Carbomer | 0,15 |
| Titanoxid | 3 |

| **Phase B** | |
|---|---|
| Triethanolamin | 0,15 |

| **Phase C** | |
|---|---|
| Siliconöl (Dimethicone) | 10 |
| Uvinul | 3 |
| Neo Heliopan | 2 |

| **Phase D** | |
|---|---|
| Camomille | 2 |
| Bsaisformulierung I | 15 |

| **Phase E** | |
|---|---|
| Parfüm | 0,3 |
| Konservierungsmittel | 0,3 |

Die Phasen A und C werden separat hergestellt und bei etwa 70 °C unter Rühren vermischt. Bei 70 °C wird die Phase C unter Rühren zugegeben. Danach wird das Gemisch auf etwa 35 °C abgekühlt, und die Phasen D und E werden unter Rühren zugegeben. Das Gemisch wird homogenisiert.

### Beispiel 4

### Bräunungscreme I

| **Phase A** | |
|---|---|
| Wasser | q.s. ad 100 |
| Glycerin | 5 |
| Propylenglycol | 8 |

| **Phase B** | |
|---|---|
| Bienenwachs | 1,5 |
| Sheabutter | 5 |
| Jojobaöl | 3 |
| Siliconöl (Cyclomethicone) | 5 |

| **Phase C** | |
|---|---|
| Basisformulierung II | 10 |

| **Phase D** | |
|---|---|
| Parfüm | 0,3 |
| Konservierungsmittel | 0,3 |

### Beispiel 5

### Bräunungscreme II

| **Phase A** | |
|---|---|
| Wasser | q.s. ad 100 |
| Glycerin | 6 |
| Propylenglycol | 9 |

| **Phase B** | |
|---|---|
| Bienenwachs | 1,7 |
| Sheabutter | 5 |
| Jojobaöl | 4 |
| Siliconöl (Cyclomethicone) | 4,5 |

| **Phase C** | |
|---|---|
| Basisformulierung I | 5 |

| **Phase D** | |
|---|---|
| Parfüm | 0,3 |
| Konservierungsmittel | 0,3 |

### Beispiel 6

### Körperlotionsgel für Bräunung

| **Phase A** | |
|---|---|
| Wasser | q.s. ad 100 |
| Glycerin | 5 |
| Carbomer | 0,3 |

| **Phase B** | |
|---|---|
| Triethanolamin | 0,3 |

| **Phase C** | |
|---|---|
| Dimethicone | 10 |
| Ethanol | 5 |

| **Phase D** | |
|---|---|
| Basisformulierung I | 8 |

| **Phase E** | |
|---|---|
| Parfüm | 0,4 |
| Konservierungsmittel | 0,2 |

### Beispiel 7

### Stabilisierungstest

Die Veränderung der DHA-Konzentration wurde chromatographisch bei Lagerung der Testproben in klimatisierter Atmosphäre bei 45 °C gemessen. Es wurden 4 Basisformulierungen auf wässriger Basis hergestellt, enthaltend

| | |
|---|---|
| Probe B | 2 % DHA |
| | 1 % Truxerutin |
| | 97 % Wasser |
| Probe C | 2 % DHA |
| | 1 % Truxerutin |
| | 0,5 % RPF-Komplex I* |
| | 0,25 % Rutinylsulfat |
| Probe E | 4 % DHA |
| | 1 % Truxerutin |
| Probe F | 4 % DHA |
| | 1 % Truxerutin |
| | 0,5 % RPF-Komplex I* |
| | 0,25 % Rutinylsuflat |

| | |
|---|---|
| * Zusammensetzung wie Beispiel 1 | |

### DHA-Konzentration

| Probe | Tag Null | | nach 2 Wochen | nach 4 Wochen | nach 8 Wochen |
|---|---|---|---|---|---|
| | Theorie gemessen | | % Veränderung | % Veränderung | % Veränderung |
| B | 2 | 2,11 | 1,99 (-5,7 %) | 1,75 (-17,1 %) | 1,52 (-28 %) |
| C | 2 | 2,01 | 1,99 (-1,0 %) | 1,95 (-3 %) | 1,88 (-6,5 %) |
| E | 4 | 3,89 | 3,81 (-2,1 %) | 3,42 (-12,1 %) | 2,92 (-25 %) |
| F | 4 | 3,98 | 3,94 (-1,1 %) | 3,72 (-6,5 %) | 3,64 (-8,5 %) |

Die Konzentrationsverringerung in den Proben B und E ist etwa dreimal so hoch wie in den Proben C und F. Wegen des geringeren DHA-Abbaus und durch den offensichtlichen Stabilisierungseffekt bei C und F ist auch eine signifikante Bräunungsverlängerung zu erwarten, was durch den Test in Beispiel 8 bestätigt wurde.

### Beispiel 8

### Bräunungstest

In einer Probandengruppe von 12 Personen mit Mischhaut wurden auf den Unterarm jedes Probanden zwei Felder von jeweils 2 cm² gekennzeichnet und darauf zwei verschiedene Bräunungscremes aufgetragen. Creme A entsprach der Zusammensetzung von Beispiel 4. Creme B enthielt anstelle der Basisformulierung II allein 1 % DHA.

Unmittelbar nach dem Auftragen wurde der erreichte Farbton mit einer Farbskala vom Tester verglichen. Die Farbskala zeigte Brauntöne von 1 bis 6, wobei 6 der dunkelste Braunton war. Die Messungen sind in Tabelle 1 aufgeführt. Der Unterarm der Testpersonen war im Testzeitraum stets bedeckt und keiner UV-Strahlung ausgesetzt.
Die Ergebnisse zeigen einen Abfall der Brauntönung bei der Creme B vom Mittelwert 5, 9 auf 4,1, während bei der Creme A ein Abfall von nur 5,9 auf 5,1 zu erkennen war.

### Erreichter Farbton nach Skala 1 - 6

| | **Start** | | **nach 2** | | **nach 4** | | **nach 8** | |
|---|---|---|---|---|---|---|---|---|
| | | | **Wochen** | | **Wochen** | | **Wochen** | |
| **Test-personen** | **A** | **B** | **A** | **B** | **A** | **B** | **A** | **B** |
| **1** | 6 | 6 | 6 | 5 | 5,5 | 4,5 | 5,5 | 4 |
| **2** | 6 | 6 | 6 | 5,5 | 5,5 | 5 | 5 | 5 |
| **3** | 6 | 5,5 | 6 | 4,5 | 6 | 4 | 4,5 | 3,5 |
| **4** | 5,5 | 6 | 5 | 5,5 | 4,5 | 4,5 | 4,5 | 4 |
| **5** | 6 | 6 | 6 | 6 | 5 | 4 | 5 | 4 |
| **6** | 6 | 6 | 5,5 | 5 | 5 | 4,5 | 5 | 3,5 |
| **7** | 6 | 5,5 | 6 | 4,5 | 5,5 | 4 | 5,5 | 4 |
| **8** | 6 | 6 | 5,5 | 5 | 5 | 5 | 5 | 4,5 |
| **9** | 6 | 6 | 6 | 5 | 5,5 | 4,5 | 5 | 4 |
| **10** | 6 | 6 | 6 | 5,5 | 6 | 4,5 | 6 | 4,5 |
| **11** | 6 | 6 | 5,5 | 5,5 | 5 | 5 | 5 | 5 |
| **12** | 6 | 6 | 5,5 | 5 | 5 | 4 | 5 | 3,5 |
| ∅ | **5,9** | **5,9** | **5,75** | **5,2** | **5,2** | **4,46** | **5,1** | **4,1** |

## Patentansprüche

1. Kosmetisches Hautbräunungsmittel auf Basis von Dihydroxyaceton (DHA) mit verbesserter Bräunungswirkung, umfassend eine Basisformulierung, die DHA, Trihydroxyethylrutin, Rutinylsulfat und mindestens ein weiteres Antioxidationsmittel enthält.

2. Hautbräunungsmittel nach Anspruch 1, **dadurch gekennzeichnet, dass** es Wasser, kosmetisch annehmbare Hilfsstoffe, Trägerstoffe, weitere kosmetische Wirkstoffe und/oder Gemische davon umfasst.

3. Hautbräunungsmittel nach Anspruch 1 oder 2, **dadurch gekennzeichnet, dass** der Anteil der Basisformulierung 0,5 bis 30 Gew-% beträgt, bezogen auf das Gesamtgewicht des Mittels.

4. Hautbräunungsmittel nach einem der Ansprüche 1 bis 3, **dadurch gekennzeichnet, dass** die Basisformulierung bis zu 24 Gew-% eines Gemisches aus DHA und Trihydroxyethylrutin enthält, vorzugsweise 6 bis 20 Gew-%.

5. Hautbräunungsmittel nach einem der Ansprüche 1 bis 4, **dadurch gekennzeichnet, dass** das Verhältnis von DHA zu Trihydroxyethylrutin im Bereich von 60-80: 40-20 liegt.

6. Hautbräunungsmittel nach einem der Ansprüche 1 bis 5, **dadurch gekennzeichnet, dass** die Basisformulierung 0,5 bis 6 Gew-% Rutinylsulfat enthält, vorzugsweise 0,5 bis 3 Gew-%.

7. Hautbräunungsmittel nach einem der Ansprüche 1 bis 6, **dadurch gekennzeichnet, dass** die Basisformulierung 2 bis 12 Gew-% Antioxidationsmittel enthält.

8. Hautbräunungsmittel nach einem der Ansprüche 1 bis 7, **dadurch gekennzeichnet, dass** das Antioxidationsmittel ausgewählt ist aus der Gruppe umfassend Vitamin C, Vitamin A, Vitamin E, Derivate von Vitamin C, Derivate von Vitamin A, Derivate von Vitamin E, Gemische der Derivate, Folsäure, Derivate von Folsäure, Flavone, Flavonoide, Aminosäuren, Derivate von Aminosäuren, Carotinoide, Carotine, Harnsäure, Derivate von Harnsäure, α-Hydroxysäuren, Pflanzenextrakte und Gemische von Pflanzenextrakten oder Gemische von mehreren Antioxidationsmitteln, vorzugsweise einen Pflanzenextrakt oder ein Gemisch von Pflanzenextrakten.

9. Hautbräunungsmittel nach einem der Ansprüche 1 bis 8, **dadurch gekennzeichnet, dass** das Antioxidationsmittel ein Gemisch von Pflanzenextrakten ist, bestehend aus
(a) einem durch Extraktion der Rinde von Quebracho blanco und nachfolgender enzymatischer Hydrolyse gewonnenem Produkt, das wenigstens 90 Gew-% Proanthocyanidin-Oligomere und höchstens 10 Gew-% Gallussäure enthält, wobei der Extrakt in einer an Mikrokapseln gebundenen Wirkstoffkonzentration von 2 Gew-% vorliegt, im Bereich von 1 bis 10 Gew-% liegt;
(b) einem durch Extraktion gewonnenen Seidenraupenextrakt, der das Peptid Cecropine, Aminosäuren und ein Vitamingemisch enthält, wobei der Gehalt von (b) im Bereich von 0,1 bis 10 Gew-% liegt;
(c) einem nichtionischen, kationischen oder anionischen HydroGel oder Gemisch von Hydro-Gelen mit einem Gehalt von (c) im Bereich von 0,1 bis 5 Gew-%;
(d) einem oder mehreren Phospholipiden mit einem Anteil von 0,1 bis 30 Gew-%;
(e) Wasser; und gegebenenfalls Cyclodextrinen, wobei die Gewichtsangaben auf das Gesamtgewicht des Antioxidationsmittels bezogen sind.

10. Hautbräunungsmittel nach einem der Ansprüche 1 bis 8, **dadurch gekennzeichnet, dass** das Antioxidationsmittel ein Gemisch von Pflanzenextrakten auf alkoholischer Basis ist, bestehend aus 0,1 bis 2 Gew-% Extrakt grüner Kaffeebohnen, 0,1 bis 2 Gew-% Extrakt von Blättern von Camellia sinensis, 0,1 bis 2 Gew-% Extrakt von Pongamia pinnata und 0,1 bis 2 Gew-% Extrakt der Wurzeln von Angelica archangelica und dem Rest bis 100 Gew-% aus einem einwertigen C₂-C₅-Alkohol, wobei es frei ist von Liposomen oder Mikrokapseln, und die Gewichtsangaben auf das Gesamtgewicht des Antioxidationsmittels bezogen sind.

## Claims

1. A cosmetic skin tanning product with an improved tanning effect based on dihydroxyacetone (DHA) and comprising a base formulation including DHA, trihydroxyethylrutin, rutinyl sulfate and at least one additional antioxidant.

2. The skin tanning product according to claim 1, **characterized in that** it comprises water, cosmetically acceptable auxiliaries, carriers, further cosmetic active ingredients and/or mixtures thereof.

3. The skin tanning product according to claim 1 or 2, **characterized in that** the proportion of said base formulation is 0.5 to 30 wt.%, based on the total weight of the product.

4. The skin tanning product according to any of claims 1 to 3, **characterized in that** the base formulation includes up to 24 wt.% of a mixture of DHA and trihydroxyethylrutin, preferably 6 to 20 wt.%.

5. The skin tanning product according to any of claims 1 to 4, **characterized in that** the ratio of DHA to trihydroxyethylrutin is in the range of 00-80: 40-20.

6. The skin tanning product according to any of claims 1 to 5, **characterized in that** the base formulation includes 0.5 to 6 wt.% rutinyl sulfate, preferably 0.5 to 3 wt.%.

7. The skin tanning product according to any of claims 1 to 6, **characterized in that** the base formulation includes 2 to 12 wt.% antioxidant.

8. The skin tanning product according to any of claims 1 to 7, **characterized in that** the antioxidant is selected from the group comprising vitamin C, vitamin A, vitamin E, derivatives of vitamin C, derivatives of vitamin A, derivatives of vitamin E, mixtures of said derivatives, folic acid, derivatives of folic acid, flavones, flavonoids, amino acids, derivatives of amino acids, carotenoids, carotenes, uric acid, derivatives of uric acid, α-hydroxy acids, plant extracts and mixtures of plant extracts or mixtures of several antioxidants, preferably a plant extract or a mixture of plant extracts.

9. The skin tanning product according to any of claims 1 to 8, **characterized in that** the antioxidant is a mixture of plant extracts consisting of
(a) a product obtained by extraction of the bark of Quebracho blanco and subsequent enzymatic hydrolysis, which product includes at least 90 wt.% proanthocyanidin oligomers and at most 10 wt.% gallic acid, the extract in the form of an active substance concentration of 2 wt.% bound to microcapsules being in the range of from 1 to 10 wt.%;
(b) a silkworm extract obtained by extraction, which includes the peptide cecropin, amino acids and a vitamin mixture, the content of (b) ranging from 0.1 to 10 wt.%;
(c) a nonionic, cationic or anionic hydrogel or mixture of hydrogels, the content of (c) ranging from 0.1 to 5 wt.%;
(d) one or more phospholipids in an amount of from 0.1 to 30 wt.%;
(e) water; and optionally cyclodextrins, the weight data being based on the total weight of antioxidant.

10. The skin tanning product according to any of claims 1 to 8, **characterized in that** the antioxidant is a mixture of alcohol-based plant extracts, consisting of 0.1 to 2 wt.% of an extract of green coffee beans, 0.1 to 2 wt.% of an extract of Camellia sinensis leaves, 0.1 to 2 wt.% of an extract of Pongamia pinnata, and 0.1 to 2 wt.% of an extract of Angelica archangelica roots and a balance of a monohydric C₂-C₅ alcohol to make 100 wt.%, and the product is free of liposomes or microcapsules, the weight data above being based on the total weight of antioxidant.

## Revendications

1. Produit cosmétique auto-bronzant à base de dihydroxyacétone (DHA) ayant un effet de bronzage amélioré, comprenant une formulation de base, qui contient du DHA, du trihydroxyéthylrutine, du rutinyl sulfate et au moins un autre agent antioxydant.

2. Produit auto-bronzant selon la revendication 1, **caractérisé en ce qu'**il contient de l'eau, des adjuvants cosmétiquement acceptables, des vecteurs, d'autres principes actifs cosmétiques et/ou des mélanges d'entre eux.

3. Produit auto-bronzant selon la revendication 1 ou 2, **caractérisé en ce que** la proportion de la formulation de base est de 0,5 à 30 % en poids, rapporté au poids total du produit cosmétique.

4. Produit auto-bronzant selon l'une quelconque des revendications 1 à 3, **caractérisé en ce que** la formulation de base contient jusqu'à 24 % en poids d'un mélange de DHA et de trihydroxyéthylrutine, de préférence de 6 à 20 % en poids.

5. Produit auto-bronzant selon l'une quelconque des revendications 1 à 4, **caractérisé en ce que** le rapport du DHA au trihydroxyéthylrutine est dans une plage de 60-80 : 40-20.

6. Produit auto-bronzant selon l'une quelconque des revendications 1 à 5, **caractérisé en ce que** la formulation de base contient 0,5 à 6 % en poids de rutinyl sulfate, de préférence 0,5 à 3 % en poids.

7. Produit auto-bronzant selon l'une quelconque des revendications 1 à 6, **caractérisé en ce que** la formulation de base contient 2 à 12 % en poids d'agent antioxydant.

8. Produit auto-bronzant selon l'une quelconque des revendications 1 à 7, **caractérisé en ce que** l'agent antioxydant est choisi dans le groupe comprenant la vitamine C, la vitamine A, la vitamine E, les dérivés de vitamine C, les dérivés de vitamine A, les dérivés de vitamine E, les mélanges des dérivés, l'acide folique, les dérivés d'acide folique, les flavones, les flavonoïdes, les acides aminés, les dérivés d'acides aminés, les caroténoïdes, les carotènes, l'acide urique, les dérivés d'acide urique, les α-hydroxyacides, les extraits de plantes et les mélanges d'extraits de plantes ou les mélanges de plusieurs antioxydants et, de préférence un extrait de plante ou un mélange d'extraits de plantes.

9. Produit auto-bronzant selon l'une quelconque des revendications 1 à 8, **caractérisé en ce que** l'agent antioxydant est un mélange d'extraits de plantes, constitué des suivants:
(a) un produit obtenu par extraction d'écorce de Quebracho blanco et hydrolyse enzymatique suivante, qui contient au moins 90 % en poids d'oligomères de proanthocyanidine et au maximum 10 % en poids d'acide gallique, l'extrait, dans une concentration de principe actif liée à des microcapsules de 2 % en poids, étant à une teneur dans une plage de 1 à 10 % en poids,
(b) un extrait de vers à soie obtenu par extraction, qui contient le peptide cécroprine, des acides aminés et un mélange de vitamines, la teneur de (b) étant dans une plage de 0,1 à 10 % en poids;
(c) un hydrogel non ionique, cationique ou anionique ou des mélanges d'hydrogels, ayant une teneur de (c) dans une plage de 0,1 à 5 % en poids,
(d) un ou plusieurs phospholipides dans une proportion de 0,1 à 30 % en poids,
(e) de l'eau, et éventuellement des cydodextrines, moyennant quoi les données en poids se rapportent au poids total de l'agent antioxydant.

10. Produit auto-bronzant selon l'une quelconque des revendications 1 à 8, **caractérisé en ce que** l'agent antioxydant est un mélange d'extraits alcooliques de plantes, constitué de 0,1 à 2 % en poids d'extrait de grains de café vert, de 0,1 à 2 % en poids d'extrait de feuilles de Camellia sinensis, de 0,1 à 2 % d'extrait de Pongamia pinnata et de 0,1 à 2 % en poids d'extrait de racines d'Angelica archangelica et le reste jusqu'à 100 % en poids d'un alcool monovalent en C₂-C₅, moyennant quoi il ne contient pas de liposomes ni de microcapsules et les données en poids se rapportent au poids total de l'agent antioxydant.
